# EUROPÄISCHE PATENTSCHRIFT

(11) **EP 3 576 648 B1**
(45) Veröffentlichungstag und Bekanntmachung des Hinweises auf die Patenterteilung: **12.08.2020**
(21) Anmeldenummer: 18703304.8
(22) Anmeldetag: 02.02.2018
(51) Int. Cl.: A61B 17/28, A61B 17/29, A61B 17/00

(54) **CHIRURGISCHES INSTRUMENT MIT EINEM LÖSEMECHANISMUS ZUR ENTKOPPLUNG EINES SCHAFTABSCHNITTS VON EINEM GRIFF**
SURGICAL INSTRUMENT HAVING A RELEASE MECHANISM FOR DECOUPLING A SHAFT SECTION FROM A HANDLE
INSTRUMENT CHIRURGICAL MUNI D'UN MÉCANISME DE DÉBLOCAGE POUR DÉSACCOUPLER UN SEGMENT DE TIGE D'UNE POIGNÉE

(30) Priorität: 03.02.2017 DE 102017102176
(43) Veröffentlichungstag der Anmeldung: 11.12.2019
(73) Patentinhaber: Aesculap AG, 78532 Tuttlingen (DE)
(72) Erfinder: SCHWEITZER, Tom, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2018/052649
(87) Internationale Veröffentlichungsnummer: WO 2018/141904

(56) Entgegenhaltungen:
- EP-A1- 2 510 888
- EP-A2- 2 653 120
- DE-A1-102004 030 928
- DE-A1-102011 001 891
- DE-B3- 10 357 105

## Beschreibung

Die vorliegende Erfindung betrifft ein chirurgisches Instrument, etwa ein chirurgisches Greif- und/oder Schneidinstrument, also allgemein Rohrschaftinstrumente, gemäß dem Oberbegriff des Anspruchs 1. Ein derartiges Instrument ist aus EP2653120A2 bekannt. Gattungsgemäße Instrumente weisen neben einem Schaftabschnitt einen Griff auf, der sich zumindest aus einem starren und einem relativ dazu beweglichen Teil zusammensetzt.

### Hintergrund der Erfindung

Wiederverwendbare chirurgische Instrumente, sei es in der minimalinvasiven Chirurgie oder in der Endoskopie, werden nach jedem Einsatz sterilisiert, desinfiziert oder zumindest gereinigt. Dieser Vorgang läuft üblicherweise in einer zentralen Sterilgutversorgungsanlage (ZSVA) ab. In solchen ZSVA wird eine Vielzahl von unterschiedlichen Instrumenten von unterschiedlich qualifizierten Fachkräften desinfiziert.

Da chirurgische Instrumente einer Bauraumoptimierung Folge leistend einen hohen Kompaktheitsgrad aufweisen, ist es in den meisten Fällen notwendig, die Instrumente in ihre Einzelteile zu zerlegen / aufzuteilen, um eine vollumfängliche Desinfektion zu gewährleisten. Der Mechanismus, der für eine Zerlegung des Instruments in seine Einzelteile verantwortlich ist, wird Lösemechanismus genannt. Bei diesem ist darauf zu achten, dass er möglichst intuitiv ausführbar ist, ein hohes Maß an Zuverlässigkeit garantiert und nicht zuletzt auch zeitlich effizient ausführbar ist.

### Stand der Technik

Aus der Deutschen Patentanmeldung DE 10 2012 007 650 A1 ist eine lösbare mechanische Kopplung zwischen einem Griff und einem Schaft offenbart, um eine Zerlegbarkeit der einzelnen Komponenten für einen Desinfektionsvorgang zu gewährleisten. In jener Patentanmeldung sind die voneinander zu entkoppelnden Komponenten darauf angewiesen, eine Entkopplungsposition einzunehmen, um die Zerlegbarkeit zu gewährleisten.

Im Stand der Technik sind allgemein Instrumente offenbart, in denen Rückstellfedern dafür sorgen, dass Entkopplungspositionen eingenommen werden. Hierbei ist jedoch zu beachten, dass jene Rückstellfedern abhängig von der Relativposition des beweglichen zum starren Griffteil sind.

Nachteilig an dem Stand der Technik ist somit, dass für eine Ver- sowie eine Entriegelung von modular verbindbaren Bauteilkomponenten stets darauf zu achten ist, dass die einzelnen Teile korrekt / in der dafür vorgesehenen Stellung zueinander stehen. Schränkt beispielsweise ein Anwender in einer ZSVA die freie Beweglichkeit eines Griffteils unabsichtlich ein, so kann dies zu einer inkorrekten Zerlegung / Entriegelung bzw. zu einem inkorrekten Zusammenbauen / Verriegeln des chirurgischen Instruments führen. Dies bewirkt im schlimmsten Fall ein fehlerhaftes chirurgisches Instrument, was besonders bei intraoperativen Ent- und Verriegelungsprozessen ein Gesundheitsrisiko für Patienten darstellen kann.

### Kurzbeschreibund der Erfindung

Der vorliegenden Erfindung liegt angesichts dieses Standes der Technik die Aufgabe zugrunde, die Nachteile aus dem Stand der Technik zu beheben oder zumindest zu mildern, und insbesondere ein chirurgisches Instrument zu offenbaren, dessen Lösemechanismus eine einfachere Montage und Demontage von Instrumentengriff und Schaft erlaubt.

Weiterhin zielt die Erfindung darauf ab, den Lösemechanismus möglichst intuitiv zu realisieren, sodass Anwender auch ohne spezifisches Wissen über das jeweilige Instrument dazu in der Lage sind, schnell ein Zerlegen sowie ein späteres Zusammenbauen durchzuführen.

Die vorliegende Erfindung strebt ebenso an, einem Anwender, der das Zusammenbauen des Griffs und des Schaftabschnitts durchführt, ein akustisches Signal / eine akustische Rückmeldung zu geben, die ihn über die korrekte Montage informiert.

Dies wird erfindungsgemäß mittels eines chirurgischen Instruments mit den Merkmalen des Anspruchs 1 gelöst. Vorteilhafte Ausführungsformen sind Gegenstand der Unteransprüche.

Aus dieser erfindungsgemäßen Ausgestaltung eines chirurgischen Instruments lassen sich somit beispielsweise folgende weitere Vorteile ableiten:
- Die Montage / Demontage erfolgt erfindungsgemäß (im Wesentlichen) unabhängig von der Griffstellung, woraus eine hohe Zuverlässigkeit sowie eine einfachere Montage und Demontage resultiert.
- Ein Anwender, etwa in einer ZSVA, kann den Lösemechanismus intuitiv bedienen und muss keine Griffstellungen beachten, woraus eine Zeitersparnis in der Desinfektion resultiert.
- Die Gebrauchstauglichkeit ist dadurch erhöht, das weniger Kriterien für eine korrekte Desinfektion zu erfüllen sind.
- Die Lebensdauer der einzelnen Instrumente ist dadurch erhöht, dass weniger Beschädigungen aufgrund inkorrekter (De-)Montageprozesse auftreten.
- Der Lösemechanismus realisiert ein Poka-Yoke-Prinzip, da sowohl eine inkorrekte Demontage, als auch eine inkorrekte Montage nicht bzw. nur unter Gewalteinwirkung möglich sind.
- Es wird gegenüber herkömmlichen Lösungen kein zusätzlicher Bauraum benötigt, da bereits verwendete Bauteile eingesetzt werden.
- Aufgrund der hohen Modularität sowie der ähnlichen Teile ist die Lösung annähernd kostenneutral umsetzbar.

Der Gegenstand der Erfindung ist demnach ein (minimal invasives) chirurgisches Instrument, wie ein Rohrschaftinstrument, mit einem Schaftabschnitt, der an seinem proximalen Endabschnitt mit einem (Instrumenten-)Griff verbunden ist, über den eine Greif- und/oder Schneide- und/oder Haltefunktion des Instruments realisierbar/aktuierbar ist. Der Griff weist hierfür ein starres Griffteil (zum Halten des Instruments) und ein dazu bewegliches Griffteil (zum Aktuieren der Instrumentenfunktionen) auf. Um die der Erfindung zugrundeliegende einfache Zerlegbarkeit (und Montage) zu gewährleisten ist weiterhin ein Lösemechanismus vorgesehen, der erfindungsgemäß so ausgebildet ist, dass bei seiner (manuellen, ggf. singulären) Betätigung der Schaftabschnitt und der Griff unabhängig von der jeweiligen aktuellen Position des beweglichen Griffteils relativ zum starren Griffteil voneinander entkoppelt / gelöst sind.

Gemäß der Erfindung weist der Lösemechanismus bevorzugt ein (einziges) Betätigungselement auf, das mittels einer (einzigen) Betätigungsbewegung, z.B. Verschwenkung, den Schaftabschnitt, der einen starren Teil und eine zu diesem bewegliche Schubstange aufweist, und auch den Griff voneinander löst / zerlegt / entriegelt / demontiert. Im Umkehrschluss ermöglicht das Betätigungselement auch ein Einstecken des Schaftabschnitts in den Griff, um nach der Desinfektion ein Sperren / Zusammenbauen / eine Montage / ein Verriegeln zu gewährleisten. Im Zentrum der Erfindung steht somit, dass der Lösemechanismus ein Betätigungselement aufweist, das mittels einer Betätigungsbewegung den aus einem starren Teil und einer beweglichen Schubstange zusammengesetzten Schaftabschnitt und den Griff in distaler Richtung vollständig voneinander löst. Hierunter ist zu verstehen, dass zwischen dem Schaftabschnitt und dem Griff keine Schnappverbindung mehr vorliegt, sodass diese Komponenten über ein Ziehen des Schaftabschnitts in Richtung distal (relativ zum Griff) frei beweglich sind und somit vollständig voneinander entkoppelbar sind.

In anderen Worten ausgedrückt zeichnet sich die Erfindung dadurch aus, dass der Lösemechanismus ein (einziges) Betätigungselement aufweist, das derart ausgeformt / beschaffen / ausgestaltet ist, dass der aus einem starren Teil und einer beweglichen Schubstange zusammengesetzte Schaftabschnitt und der Griff in distaler Richtung vollständig voneinander lösbar sind.

In einer vorteilhaften Ausführungsform sind durch die Verschwenkung des (einzigen) Betätigungselements der Schaftabschnitt und der Griff voneinander unabhängig von der Position des beweglichen Griffteils relativ zum starren Griffteil in distaler Richtung gelöst. Das bedeutet, dass ein Anwender, der die Demontage des Schaftabschnitts von dem Griff vornimmt, keinerlei zusätzliche Vorkehrungen zu treffen hat, um deren Richtigkeit sicherzustellen. Insbesondere ist nicht darauf zu achten, wie das bewegliche Griffteil relativ zum starren Griffteil steht. Hieraus folgt eine hohe Betriebssicherheit nicht nur in der Demontage sondern auch in der Montage, die am Ende des Desinfektionszyklus steht.

Das Betätigungselement ist vorzugsweise wippenartig, also nach Art einer Wippe, ausgestaltet. Das heißt es weist einen proximalen und einen distalen Abschnitt auf, die zueinander um einen Drehpunkt verschwenkbar sind. Im weiteren Verlauf wird von dem wippenartigen Betätigungselement gesprochen, wobei die Wippenartigkeit nicht zwingend erforderlich, sondern lediglich in einer bevorzugten Ausführungsform beansprucht ist. So wäre es beispielsweise auch möglich, das Betätigungselement als Zug- oder Druckknopf auszubilden, das z.B. längsverschiebbar im starren Griffteil gelagert ist und im Verlauf seiner Betätigungsbewegung zwei Verriegelungs-/Rastmechanismen, den einen zur Kopplung vom Schaftabschnitt mit dem starren Griffteil und den anderen zur Kopplung eines Bewegungsübertragungselements im Schaftabschnitt mit dem beweglichen Griffteil, parallel oder seriell entriegelt. Hierbei sei darauf hingewiesen, dass ein solcher Zug- oder Druckknopf auch durch einen einfachen Schwenkhebel ersetzt sein könnte, der im Verlauf seiner Schwenkbewegung die beiden Verriegelungs-/Rastmechanismen entriegelt.

Von Vorteil ist es auch, wenn durch die eine Verschwenkung des Betätigungselements sowohl eine erste Verbindung zwischen dem starren Griffteil und dem starren Teil des Schaftabschnitts als auch eine zweite Verbindung zwischen dem beweglichen Griffteil und der beweglichen Schubstange des Schaftabschnitts in distaler Richtung gelöst sind. Bei einer Verschwenkung wird ein Endabschnitt des verschwenkten Teils in die eine Richtung und der andere Endabschnitt in die andere Richtung bewegt. Vorliegend wird sich dieser Effekt also zunutze gemacht: der in die eine Richtung verschwenkte Teil des Betätigungselements löst eine Rastnase aus dem starren Teil ("erste Verbindung"), der in die andere Richtung verschwenkte Teil des Betätigungselements drückt einen Mitnahmestift aus einer Öse / Einbuchtung ("zweite Verbindung"), sodass mittels einer Verschwenkung zwei Verbindungen gelöst werden, wodurch die erfindungsgemäße zweifache Wirkung des Betätigungselements effizient erreichbar ist.

Eine weitere Ausführungsform des chirurgischen Instruments zeichnet sich dadurch aus, dass durch die (einzige) Verschwenkung des vorzugsweise wippenartigen Betätigungselements gleichzeitig oder nacheinander im Verlauf dieser Betätigungsbewegung/Verschwenkung eine erste Verbindung/Kopplung zwischen dem starren Griffteil und einem (starren) Teil des Schaftabschnitts und eine zweite Verbindung zwischen dem beweglichen Griffteil und einer beweglichen Schubstange (Bewegungsübertragungselement) des Schaftabschnitts gelöst sind. So ist nur eine (einzige) Bewegung des bevorzugt wippenartigen Betätigungselements nötig, um beide Verbindungen voneinander zu lösen, was sowohl den Entkopplungs- als auch den Verriegelungsprozess beschleunigt.

Weiterhin ist es vorteilhaft, wenn das Betätigungselement eine (bezüglich des Griffs/Griffgehäuses) nach außen zeigende Betätigungsfläche aufweist, die über eine, vorzugsweise lineare, Druckbewegung die Verschwenkung des (wippenartigen) Betätigungselements hervorruft und somit die Lösbarkeit des Schaftabschnitts von dem Griff sicherstellt. Eine solche Druckbewegung, die verglichen mit der Höhe des Instruments nur über einen sehr kurzen Weg zurückzulegen ist, stellt einen intuitiv verständlichen Lösemechanismus auch für unerfahrene Anwender dar. Eine lineare und zudem kurze Druckbewegung ist weiterhin verschleißarm in das System integrierbar, was dessen Lebensdauer erhöht.

Eine hochkompakte Ausführungsform ist bevorzugt dadurch realisiert, dass die Betätigungsfläche des Betätigungselements in einem Verriegelungszustand zwischen dem Griff und dem Schaftabschnitt im Wesentlichen bündig zu einer Außenfläche des starren Griffteils (Griffgehäuses) verläuft. So weist das bevorzugt wippenartige Betätigungselement in der Seitenansicht im Wesentlichen eine Dreiecksform / eine Keilform mit einem rechten Winkel auf, deren Hypotenuse sich planparallel / komplementär / bündig mit der entsprechenden Außenseite des starren Griffteils erstreckt. Unter dem Verriegelungszustand wird der Zustand verstanden, in dem das Betätigungselement vorgespannt seine Ausgangsstellung einnimmt und den Griff mit dem Schaftabschnitt derart miteinander koppelt / verbindet, dass das chirurgische Instrument einsatzbereit /-fähig ist. Um vor dem Hintergrund der Bündigkeit der Flächen überhaupt eine Druckbewegung / Verschwenkung des bevorzugt wippenartigen Betätigungselements zu ermöglichen, ist ein Teil des starren Griffteils, mit einer Aussparung versehen, die ein Eindrücken der Betätigungsfläche und somit eine Verschwenkung des bevorzugt wippenartigen Betätigungselements sicherstellt.

Es ist zudem vorteilhaft, wenn das bevorzugt wippenartige Betätigungselement in seinem distalen Bereich, der im Falle der Keilform des Betätigungselement einen spitzen Winkel ausformt, einen Rastvorsprung aufweist, der dazu vorbereitet / angepasst ist, im Verriegelungszustand einen Formschluss mit einer Aufnahmetasche des Schaftabschnitts einzugehen. Jener Formschluss stellt die eingangs erwähnte erste Verbindung dar. Der Rastvorsprung ist in seiner Kontur variabel ausgestaltbar und auf die Bauraum- und Kraftanforderungen anpassbar. Der Formschluss zeichnet sich durch eine hohe Robustheit aus, wodurch eine betriebssichere Ausgestaltung des Lösemechanismus realisiert ist. Der Rastvorsprung und seine Gegenkontur in der Aufnahmetasche sind vor allem in Axialrichtung möglichst mit geringen Toleranzen zu fertigen, um den Schaftabschnitt spielfrei mit dem Griff zu verbinden.

In einer weiteren Ausführungsform ist das Betätigungselement über eine von dem starren Griffteil ausgeformte Blattfeder vorgespannt. Diese stellt sicher, dass das bevorzugt wippenartige Betätigungselement in dem Verriegelungszustand den Schaftabschnitt und den Griff zuverlässig verbindet. Die Blattfeder zeichnet sich durch ihren geringen Bauraumbedarf sowie ihre wirtschaftliche Fertigung aus. Weiterhin gewährleistet die erfindungsgemäße Anordnung der Blattfeder eine ausreichend große Kontaktfläche zwischen dem Betätigungselement und der Blattfeder, wodurch die Vorspannung über die gesamte Lebensdauer garantiert ist. Die von dem Griffteil ausgeformte Blattfeder ist sowohl als integraler Bestandteil des starren Griffteils, als auch als zusätzliche Komponente realisierbar.

Das bewegliche Griffteil weist vorteilhafterweise weiterhin einen Mitnahmestift auf, der dazu vorbereitet / angepasst ist, über eine Einbuchtung in einer zumindest teilweise innerhalb des Schaftabschnitts angeordneten Schubstange einen Formschluss zwischen dem beweglichen Griffteil und der Schubstange herzustellen, um eine Rotation des beweglichen Griffteils in eine Translation der Schubstange zu übertragen. Jener Formschluss stellt die eingangs erwähnte zweite Verbindung dar. Der Mitnahmestift ist etwa über eine Schraubenfeder vorgespannt, um so in jedem Betriebszustand mit dem Betätigungselement in Kontakt zu stehen. Sobald die Druckbewegung auf das bevorzugt wippenartige Betätigungselement ausgeführt wird, wird somit unter Bewahrung des Kontaktes sichergestellt, dass sich der Mitnahmestift aus der Einbuchtung der Schubstange löst, wodurch der Schaftabschnitt und der Griff voneinander entkoppelbar sind. Erfindungsgemäß führt die Druckbewegung des Betätigungselements in jeder Stellung des beweglichen Griffteils relativ zum Starrengriffteil zu einer Lösbarkeit / Zerlegbarkeit des Instruments.

Es ist darüber hinaus von Vorteil, wenn das bevorzugt wippenartige Betätigungselement eine Anlagefläche aufweist, die dazu vorbereitet / angepasst ist, mit dem Mitnahmestift derart in Kontakt zu stehen, dass dieser unabhängig von der Position / Rotation des beweglichen Griffteils aus der Einbuchtung lösbar ist. Das Zusammenspiel zwischen dem vorgespannten Mitnahmestift und der im Vergleich zu diesem in der Seitenansicht langen Anlagefläche stellt sicher, dass auch dann, wenn das bewegliche Griffteil gerade die Schubstange voll ausgefahren hat, noch ein Kontakt herrscht, der die Lösbarkeit des Griffs von dem Schaftabschnitt mittels der Druckbewegung des Betätigungsteils gewährleistet. Die Anlagefläche weist eine hohe Oberflächenglattheit auf, um die bei einer Verschiebung der Schubstange auftretende Reibung zwischen dem Mitnahmestift und der Anlagefläche gering zu halten.

Insbesondere dann, wenn der Rastvorsprung des bevorzugt wippenartigen Betätigungselements derart in die Aufnahmetasche des Schaftabschnitts eingreift, dass bei dem Zustandekommen des Formschlusses durch das Einschnappen ein akustisches Signal auftritt, das einem Anwender den Verriegelungszustand signalisiert, ist die Handhabung des chirurgischen Instruments auch für unerfahrene Anwender intuitiv und betriebssicher ermöglicht. Das akustische Signal stellt etwa ein Klick-Geräusch dar, das aufgrund des Einschnappens des Rastvorsprungs in die Aufnahmetasche auftritt. So bewirkt die das Betätigungselement vorspannende Blattfeder, dass die Stirnseite des Rastvorsprungs derart auf den Grund der Aufnahmetasche schlägt, dass ein Anwender ein Klick-Geräusch wahrnimmt.

Eine weitere vorteilhafte Ausführungsform der Erfindung zeichnet sich dadurch aus, dass das nunmehr wippenartige Betätigungselement um eine Schwenkachse gelagert ist, die mit dem starren Griffteil verbunden ist und die auf der dem beweglichen Griffteil abgewandten Seite der Schubstange angeordnet ist. Die Schwenkachse verläuft vorzugsweise versetzt zum Flächenmittelpunkt einer Seitenfläche des Betätigungselements, um die aufzubringende Kraft für die Druckbewegung auf das Betätigungselement über die Hebelwirkung zu minimieren. Die Schwenkachse stellt in diesem bevorzugten Fall also die Wippenfunktion / die Wippenartigkeit des Betätigungselements sicher.

Weiterführend sei offenbart, dass auch solche chirurgischen Instrumente, die mittels eines Drehsterns zu betätigen sind, mit dem erfindungsgemäßen Lösemechanismus auszustatten sind. Auch hier ist es möglich, mittels eines einzelnen Betätigungselementes die Verbindung zwischen dem festen / starren Griffteil und dem Schaftabschnitt sowie zwischen der Schubstange und dem beweglichen Griffteil zu lösen. In einem solchen Fall ist die Verriegelung statt über einen Formschluss, über eine gefederte Spannzangenverbindung erreicht. Mittels des in diesem Ausführungsbeispiel axial verschiebbaren Betätigungselements werden hierbei ebenfalls der Griff und der Schaftabschnitt unabhängig von der Griffstellung, also der Relativposition des beweglichen Griffteils zum starren Griffteil, entkoppelt.

Wie aus der vorstehenden Erläuterung der vorliegenden Erfindung zu entnehmen ist, lässt sich eine besonders bevorzugte Ausführungsform der Erfindung auf das folgende Konstruktionsprinzip reduzieren:
Insbesondere von einem wippenartigen Betätigungselement ist es allgemein bekannt, dass bei einem Drücken auf das eine Wippenende sich dieses Wippenende z.B. absenkt bzw. in ein Gehäuse eintaucht, wohingegen sich das andere Wippenende z.B. anhebt bzw. aus einem Gehäuse heraustritt. Diese Funktion macht sich die vorliegende Erfindung bevorzugt zunutze, indem das ebenfalls bevorzugt wippenartige Betätigungselement des erfindungsgemäßen Lösemechanismus so ausgebildet und am starren Griffteil (Griffgehäuse) positioniert/gelagert ist, dass dessen eine (zu drückende) Wippenende bei einer drückenden Betätigung (taucht dann in das Griffgehäuse ein) unmittelbar oder mittelbar gegen ein Kopplungselement (Mitnahmestift) drückt, welches das bewegliche Griffteil (Betätigungshebel/Betätigungsknopf/etc.) mit einem Kraft-/Bewegungsübertragungsbauteil (Schubstange) innerhalb des Instrumentenschafts koppelt, um dadurch diese Kopplung zu lösen (im Konkreten wird dadurch der federvorgespannte Mitnahmestift aus der in der Schubstange ausgebildeten Einbuchtung/Nut gedrückt), wohingegen das andere Wippenende, an welchem sich vorzugsweise z.B. eine Rastnase, ein Haken oder eine Öse befindet/ausbildet, die mit einem entsprechenden Hinterschnitt oder Vorsprung am Instrumentenschaft zusammenwirkt, aus einer den Instrumentenschaft an dem starren Griffteil (Griffgehäuse) haltenden/verriegelnden Position in eine Freigabeposition geschwenkt wird (tritt dann aus dem Griffgehäuse heraus), wodurch der Instrumentenschaft von Griff freigegeben/lösbar ist.

Auf diese Weise lassen sich grundsätzlich mit ein und demselben Betätigungselement des Lösemechanismus bei einer einzigen Betätigungsbewegung sowohl der Instrumentenschaft vom starren Griffteil, wie auch die im Instrumentenschaft gelagerte Schubstange vom beweglichen Griffteil entkoppeln/entriegeln, sodass der Instrumentenschaft samt darin gelagerter Schubstange vom Griff gelöst/demontiert/abgenommen werden kann. Die Montage des Instrumentenschafts samt darin gelagerter Schubstange erfolgt dementsprechend.

Insbesondere vorteilhaft ist es außerdem, wenn das eine Betätigungselement zweifach auf den starren Teil und die Schubstange wirkt. Folglich dient das eine Betätigungselement als zweifache Riegelvorrichtung.

In dieser Ausführungsform bildet das Betätigungselement zum ersten an seinem distalen Ende eine zum starren Teil hin ragende Rastnase und zum zweiten proximal zu dieser Rastnase eine auf einem Mitnahmestift aufliegende Anlagefläche aus, wobei bei einer Betätigung des Betätigungselements die Rastnase aus dem starren Teil ausrastet und die Anlagefläche teilweise am Mitnahmestift abgleitet. Das Betätigungselement wird bei einer Betätigung verschwenkt. Die hieraus resultierende Aufwärtsbewegung des distalen Endes des Betätigungselements wird ebenso für das Lösen eines Formschlusses verwendet, wie die Abwärtsbewegung des proximalen Endes.

In anderen Worten kann gesagt werden, dass das Betätigungselement zweifach wirkt: Erstens, es löst den Formschluss zwischen der Rastnase (des Betätigungselements) und der Aufnahmetasche (des starren Teils) und zweitens, es löst über die Anlagefläche (des Betätigungselements) den Formschluss zwischen dem Mitnahmestift (des beweglichen Griffteils) und der Einbuchtung (der Schubstange des Schaftabschnitts).

Nachfolgend sei die Erfindung anhand eines bevorzugten Ausführungsbeispiels unter Bezugnahme auf die begleitenden Figuren näher beschrieben. Es zeigen:
Fig. 1: ein proximales Ende eines erfindungsgemäßen chirurgischen Instruments in einem Verriegelungszustand;
Fig. 2: die Ansicht aus Fig. 1, wobei eine Verschwenkung eines Betätigungselements hervorgerufen worden ist; und
Fig. 3: einen von einem Griff entkoppelten Schaftabschnitt des chirurgischen Instruments.

Die Figuren sind lediglich schematischer Natur und dienen ausschließlich dem Verständnis der Erfindung. Die gleichen Elemente sind mit denselben Bezugszeichen versehen.

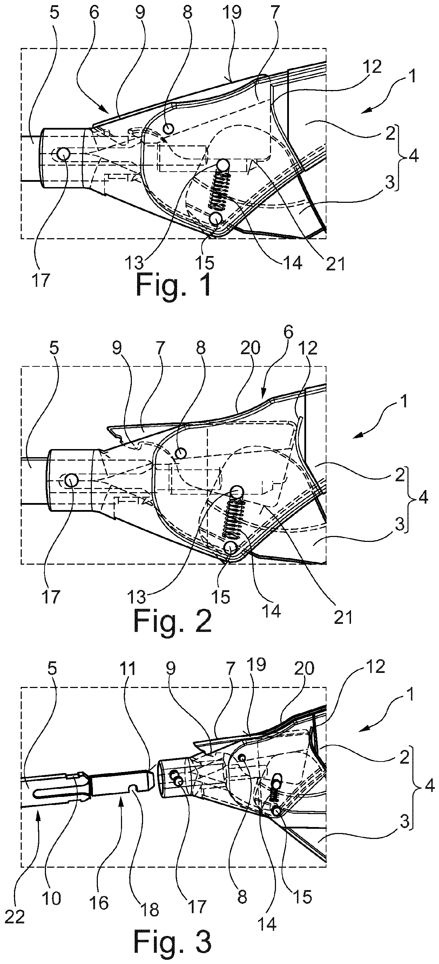

In Fig. 1 ist ein proximales Ende eines chirurgischen Instruments 1 dargestellt. Ein Schaftabschnitt/Instrumentenschaft 5 setzt sich, wie in Zusammenhang mit Fig. 3 näher erläutert, aus einem in Axialrichtung sich erstreckenden, starren oder biegeflexiblen Teil (Rohrmantel) 22 und einer darin gelagerten, verschiebbaren Schubstange 16 zusammen. Der Schaftabschnitt 5 ist an seinem proximalen Endabschnitt mit einem Instrumenten-Griff 4 verbunden. Dieser weist ein starres Griffteil (Griffgehäuse) 2 und ein dazu bewegliches Griffteil (Betätigungshebel) 3 auf.

Der Griff 4 und der Schaftabschnitt 5 (samt darin gelagerter Schubstange) sind miteinander über einen Lösemechanismus 6 mechanisch gekoppelt. Dieser ist derart betätigbar, dass der Griff 4 und der Schaftabschnitt 5 (samt darin gelagerter Schubstange) voneinander entkoppelt und getrennt werden können. Erfindungsgemäß weist der Lösemechanismus 6 hierfür ein wippenartiges Betätigungselement 7 auf, das von Außerhalb des starren Griffteils 2 frei für dessen manuelle Betätigung zugänglich ist. Dieses ist um eine Schwenkachse 8, die in dem starren Griffteil 2 gelagert ist, verschwenkbar / rotierbar. Die Verschwenkung des wippenartigen Betätigungselements 7 wird durch eine Druckbewegung von einem Anwender über eine nach außen zeigende Betätigungsfläche 19 des Betätigungselements an dessen einem Wippenende hervorgerufen.

In Fig. 1 ist das chirurgische Instrument 1 in einem Verriegelungszustand dargestellt. In diesem sind der Griff 4 und der Schaftabschnitt 5 (samt darin gelagerter Schubstange) miteinander formschlüssig gekoppelt. D.h. der Rohrmantel (Instrumentenschaft) des Schaftabschnitts 5 ist mit dem starren Griffteil 2 fest verbunden, wohingegen die Schubstange 16 mit dem beweglichen Griffteil 3, über einen Kopplungsmechanismus oder ein Kopplungselement wirkverbunden ist Sobald ein Anwender, wie ein Mitarbeiter in einer ZSVA, das Betätigungselement 7 des Lösemechanismus für dessen Verschwenken betätigt, bewirkt dies zweierlei:
- Zum ersten wird ein erster Formschluss zwischen einem betätigungselementseitigen Rastvorsprung 9 und einer im Zusammenhang mit Fig. 3 näher erläuterten schaftabschnittseitigen Aufnahmetasche/Nut 10 im Rohrmantel des Schaftabschnitts 5 gelöst. Dies entkoppelt das starre Griffteil (Griffgehäuse) 2 von dem Schaftabschnitt 5.
- Zum zweiten wird zeitgleich oder seriell in weiteren Verlauf der Betätigungsbewegung des Betätigungselements 7 ein zweiter Formschluss zwischen einem Mitnahmestift (Kopplungselement) 13 und einer ebenfalls im Zusammenhang mit Fig. 3 näher erläuterten Einbuchtung/Nut 18 in der Schubstange 16 gelöst. Dies entkoppelt das bewegliche Griffteil 3 von der in dem Schaftabschnitt 5 gelagerten Schubstange 16.

Das wippenartige Betätigungselement 7 ist über seine nach außen zeigende Betätigungsfläche 19 manuell aktuierbar. Diese ist in der in Fig. 1 dargestellten Seitenansicht im Wesentlichen bündig mit der die Betätigungsfläche randseitig umgebenden Außenfläche des starren Griffteils 2. Zudem ist die Betätigungsfläche 19 in ausreichendem Abstand von einer nicht dargestellten Grifffläche des starren Griffteils (Griffgehäuses) 2 und des beweglichen Griffteils 3 entfernt, um den über das Betätigungselement 7 auslösbaren Lösemechanismus 6 während der Benutzung des chirurgischen Instruments 1 verriegelt zu halten. Vorzugsweise ist das Betätigungselement an der als Oberseite des starren Griffteils geplanten Seite angeordnet, wohingegen sich der bewegliche Griffteil 3 an der als Unterseite des starren Griffteils geplanten Seite befindet.

Das wippenartige Betätigungselement 7 wird über eine sich am starren Griffteil abstützende Blattfeder 12 derart vorgespannt, dass in einem unbetätigten Zustand der Verriegelungszustand herrscht (Konstruktionslage). Die Blattfeder 12 wirkt dabei in Richtung des Schaftabschnitts 5 und bewirkt ein Drehmoment auf das Betätigungselement in Richtung hin zur Konstruktionslage. Die Blattfeder 12 weist vorzugsweise eine im Hinblick auf das Betätigungselement 7 konvexe Krümmung auf. Diese bewirkt unabhängig von der Vorspannung der Blattfeder 12 einen Linienkontakt zwischen der Blattfeder 12 und dem Betätigungselement 7. Die Blattfeder 12 erstreckt sich im Wesentlichen quer zur Längsrichtung des starren Griffteils 2 über dessen gesamte Breite.

Der Mitnahmestift 13 ist Teil des beweglichen Griffteils 3 oder eines zu diesem nachgeschalteten Kopplungsmechanismus (nicht dargestellt). Dieses wird bei Betätigung um eine Rotationsachse 15, die Teil des starren Griffteils 2 ist, gedreht. Es ist relativ zum starren Griffteil 2 somit verdrehbar, aber nicht verschiebbar gelagert. Der Mitnahmestift 13 ist über eine Druckfeder (Schraubenfeder) 14 in Richtung Einrastposition vorgespannt. Weiterhin ist der Mitnahmestift 13 in einem Langloch, das von dem beweglichen Griffteil 3 ausgeformt ist, gelagert. Dieses Langloch ermöglicht eine Relativbewegung zwischen dem Mitnahmestift 13 und dem beweglichen Griffteil 3.

Wird beispielsweise das bewegliche Griffteil 3 um die Rotationsachse 15 herum betätigt, so führt der Mitnahmestift 13 eine Bewegung in Axialrichtung (entspricht des Längsrichtung des Schaftabschnitts) durch. So wird die Rotation des beweglichen Griffteils 3 mittels des in dem Langloch aufgenommenen Mitnahmestifts 13 in eine Translation der Schubstange 16 gewandelt. Dadurch, dass der Mitnahmestift 13 über den zweiten Formschluss mit der Einbuchtung 18 in der Schubstange 16 gekoppelt ist, wird seine Bewegung auf die Schubstange 16 übertragen. So wird eine Betätigung des chirurgischen Instruments 1 bewirkt. Der Lösemechanismus 6 bleibt hiervon unberührt.

Über eine Anlagefläche 21 steht der Mitnahmestift 13 im vorliegenden Ausführungsbeispiel in unmittelbarem Kontakt mit dem wippenartigen Betätigungselement 7. Die Anlagefläche 21 verläuft in der Seitenansicht parallel zu der Bewegungsrichtung der Schubstange 16. Somit wird die Reibung zwischen der Schubstange und der Anlagefläche des Betätigungselements bei einer Betätigung der Schubstange 16 minimal gehalten.

Der erste Formschluss, das heißt der Formschluss zwischen dem Rastvorsprung 9 und der Aufnahmetasche 10, koppelt den Schaftabschnitt 5 und den Griff 4 (starrer Griffteil) in Axialrichtung (Längsrichtung des Schaftabschnitts). Quer zur Axialrichtung wirkt ein weiterer Formschluss zwischen dem Schaftabschnitt 5 und dem starren Griffteil 2. So weist der Schaftabschnitt 5 entlang seiner Axialrichtung eine Spur (Längsnut/Längsschlitz) auf, in die ein griffseitiger Stift 17 gleitend eingreift. Dieser ist starr mit dem starren Griffteil 2 verbunden.

In Fig. 2 ist ein zweiter Zustand, nämlich ein Verschwenkzustand des Betätigungselements, dargestellt. In diesem wird eine Verschwenkung des wippenartigen Betätigungselements 7 durchgeführt. Mittels einer Druckbewegung auf die Betätigungsfläche 19 rotiert das Betätigungselement somit um die Verschwenkachse 8. Die Druckbewegung ist durch eine Aussparung 20 im starren Griffteil 2 ermöglicht. Die Verschwenkung bewirkt eine Bewegung des Mitnahmestifts 13 in seinem Langloch für ein Ausrücken des Mitnahmestifts aus der in der Schubstange ausgebildeten Einbuchtung/Nut. Somit ist unabhängig von der Position des beweglichen Griffteils 3 relativ zum starren Griffteil 2 ein Lösen des zweiten Formschlusses, das heißt des Formschlusses zwischen dem Mitnahmestift 13 und der Einbuchtung 18 erreicht. Die Aussparung 20 ist hierbei vorzugsweise mit einer konkaven Krümmung ausgestaltet. Sie weist eine solche Form auf, dass sie ergonomisch auf die Form des Daumens eines Anwenders passt.

Zeitgleich löst sich der Rastvorsprung 9 aus der Aufnahmetasche 10, was den starren Teil 22 des Schaftabschnitts 5 von dem starren Griffteil 2 entkoppelt. Bei der Druckbewegung auf die Betätigungsfläche 19 wird das wippenartige Betätigungselement 7 gegen die Blattfeder 12 gedrückt. Die aufzubringende Kraft für eine Entkopplung / Zerlegung des chirurgischen Instruments 1 ist somit zumindest über die Blattfeder 12 steuerbar.

Alle weiteren Komponenten aus Fig. 2 sind bereits anhand der Fig. 1 beschrieben.

In Fig. 3 ist ein dritter Zustand, nämlich ein Entkoppelungszustand, dargestellt. In diesem sind der Schaftabschnitt 5 samt darin lagernder Schubstange 16 und der Griff 4 voneinander entkoppelt und getrennt, sodass eine gründliche Desinfektion der einzelnen Komponenten vorzunehmen ist.

Im Entkopplungszustand ist die Form des Schaftabschnitts 5 gut erkennbar. Die Schubstange 16 ragt proximal aus dem starren Teil 22 des Schaftabschnitts 5 heraus. An der proximalen Spitze der Schubstange 16 ist eine Einführschräge 11 dargestellt, die ein sanftes Einschieben der Schubstange 16 in den Griff 4, insbesondere an dem Rastvorsprung 19 des Betätigungselements 7 vorbei, ermöglicht. Wird der Schaftabschnitt 5 also erneut in den Griff eingeschoben, das heißt, wird das chirurgische Instrument vom Entkopplungszustand in den Verriegelungszustand überführt, so wird das von der Blattfeder 12 federnd gelagerte Betätigungselement 7 von der Einführschräge 11 und dem weiteren Profil der Schubstange 16 ausgelegt, bevor der Rastvorsprung 9 des Betätigungselements 7 in die Aufnahmetasche 10 einschnappt.

Der erste und der zweite Formschluss, das heißt der Formschluss zwischen dem Rastvorsprung 9 und der Aufnahmetasche 10 sowie der zwischen dem Mitnahmestift 13 und der Einbuchtung 18 Geometrie flexibel ausgestaltbar. Aufgrund der Zinnenform des Rastvorsprungs 9 ist es vorteilhaft, wenn die Aufnahmetasche 10 im Wesentlichen kastenförmig ausgestaltet ist. Aufgrund der Zylinderform des Mitnahmestifts 13 hingegen ist es für einen effizienten zweiten Vorsprung vorteilhaft, wenn die Einbuchtung 18 bogenförmig ausgestaltet ist.

Alle weiteren Komponenten aus Fig. 3 sind bereits anhand der Figuren 1 und 2 besch rieben.

## Patentansprüche

1. Chirurgisches Instrument (1) mit einem aus einem starren Teil (22) und einer beweglichen Schubstange (16) zusammengesetzten Schaftabschnitt (5), der an seinem proximalen Endabschnitt mit einem Griff (4) verbunden ist, welcher ein starres Griffteil (2) und ein dazu bewegliches Griffteil (3) aufweist,
sowie mit einem Lösemechanismus (6), der derart betätigbar ist, dass der Schaftabschnitt (5) und der Griff (4) voneinander entkoppelt sind,
**dadurch gekennzeichnet, dass** der Lösemechanismus (6) ein einziges Betätigungselement (7) aufweist, das mittels einer bevorzugt einzigen Betätigungsbewegung, vorzugsweise Verschwenkung, eine erste Verbindung zwischen dem starren Griffteil (2) und dem starren Teil (22) des Schaftabschnitts (5) und eine zweite Verbindung zwischen dem beweglichen Griffteil (3) und der beweglichen Schubstange (16) des Schaftabschnitts (5) löst, sodass der Schaftabschnitt (5) und der Griff (4) über ein Ziehen des Schaftabschnitts (5) in Richtung distal relativ zum Griff (4) frei beweglich sind.

2. Chirurgisches Instrument (1) nach Anspruch 1, **dadurch gekennzeichnet, dass** durch die Betätigungsbewegung, vorzugsweise Verschwenkung, des Betätigungselements (7) der Schaftabschnitt (5) und der Griff (4) voneinander unabhängig von der Position des beweglichen Griffteils (3) relativ zum starren Griffteil (2) in distaler Richtung vollständig gelöst sind.

3. Chirurgisches Instrument (1) nach einem der Ansprüche 1 oder 2, **dadurch gekennzeichnet, dass** das Betätigungselement (7) wippenartig ausgeformt ist.

4. Chirurgisches Instrument (1) nach einem der Ansprüche 1 bis 3, **dadurch gekennzeichnet, dass** durch die eine Verschwenkung des Betätigungselements (7) sowohl eine erste Verbindung zwischen dem starren Griffteil (2) und dem starren Teil (22) als auch eine zweite Verbindung zwischen dem beweglichen Griffteil (3) und der beweglichen Schubstange (16) in distaler Richtung gelöst sind.

5. Chirurgisches Instrument (1) nach einem der Ansprüche 1 bis 4, **dadurch gekennzeichnet, dass** das Betätigungselement (7) eine nach außen zeigende Betätigungsfläche (19) aufweist, die über eine Druckbewegung die Verschwenkung des Betätigungselements (7) hervorruft und somit die Lösbarkeit des Schaftabschnitts (5) von dem Griff (4) sicherstellt.

6. Chirurgisches Instrument (1) nach Anspruch 5, **dadurch gekennzeichnet, dass** die Betätigungsfläche (19) in einem Verriegelungszustand zwischen dem Griff (4) und dem Schaftabschnitt (5) im Wesentlichen bündig zu einer Außenfläche des starren Griffteils (2) verläuft.

7. Chirurgisches Instrument (1) nach einem der Ansprüche 1 bis 6, **dadurch gekennzeichnet, dass** das Betätigungselement (7) einen Rastvorsprung (9) aufweist, der dazu vorbereitet ist, im Verriegelungszustand einen Formschluss mit einer Aufnahmetasche (10) des starren Teils (22) einzugehen.

8. Chirurgisches Instrument (1) nach einem der Ansprüche 1 bis 5, **dadurch gekennzeichnet, dass** das Betätigungselement (7) im Verriegelungszustand über eine von dem starren Griffteil (2) ausgeformte Blattfeder (12) vorgespannt ist.

9. Chirurgisches Instrument (1) nach einem der einem der Ansprüche 4 bis 8, **dadurch gekennzeichnet, dass** das bewegliche Griffteil (3) einen Mitnahmestift (13) aufweist, der dazu vorbereitet ist, über eine Einbuchtung (18) in der Schubstange (16) einen Formschluss zwischen dem beweglichen Griffteil (3) und der Schubstange (16) herzustellen, um eine Rotation des beweglichen Griffteils (3) in eine Translation der Schubstange (16) zu übertragen.

10. Chirurgisches Instrument (1) nach Anspruch 9, **dadurch gekennzeichnet, dass** das Betätigungselement (7) eine Anlagefläche (21) aufweist, die dazu vorbereitet ist, mit dem Mitnahmestift (13) derart in Kontakt zu stehen, dass dieser unabhängig von der Position des beweglichen Griffteils (3) aus der Einbuchtung (18) lösbar ist.

11. Chirurgisches Instrument (1) nach einem der Ansprüche 7 bis 10, **dadurch gekennzeichnet, dass** der Rastvorsprung (9) derart in die Aufnahmetasche (10) eingreift, dass bei dem Zustandekommen des Formschlusses durch das Einschnappen ein akustisches Signal auftritt, das einem Anwender den Verriegelungszustand signalisiert.

12. Chirurgisches Instrument (1) nach einem der vorstehenden Ansprüche, **dadurch gekennzeichnet, dass** das eine Betätigungselement (7) zweifach auf den starren Teil (22) und die Schubstange (16) wirkt.

13. Chirurgisches Instrument (1) nach Anspruch 12, **dadurch gekennzeichnet, dass** das Betätigungselement (7) an seinem distalen Ende eine zum starren Teil (22) hin ragende Rastnase (9) ausbildet und proximal zu dieser eine auf einem Mitnahmestift (13) aufliegende Anlagefläche (21) ausbildet, wobei bei einer Betätigung des Betätigungselements (7) die Rastnase (9) aus dem starren Teil (22) ausrastet und die Anlagefläche (21) teilweise am Mitnahmestift (13) abgleitet.

## Claims

1. A surgical instrument (1) having a shaft section (5), composed of a rigid part (22) and a movable push rod (16), which is connected on the proximal end section thereof to a handle (4), which has a rigid handle part (2) and a handle part (3) which is movable relative thereto,
and having a release mechanism (6) which can be actuated such that the shaft section (5) and the handle (4) are decoupled from each other,
**characterized in that** the release mechanism (6) comprises one single actuating element (7) which, preferably by means of one single actuating movement, preferably a swiveling movement, releases a first connection between the rigid handle part (2) and the rigid part (22) of the shaft section (5) and a second connection between the movable handle part (3) and the movable push rod (16) of the shaft section (5), so that the shaft section (5) and the handle (4) are freely movable by pulling the shaft section (5) in a distal direction relative to the handle (4).

2. The surgical instrument (1) according to claim 1, **characterized in that** the actuating movement, preferably swiveling movement, of the actuating element (7) causes the shaft section (5) and the handle (4) to be completely released in a distal direction irrespectively of the position of the movable handle part (3) relative to the rigid handle part (2).

3. The surgical instrument (1) according to one of claims 1 or 2, **characterized in that the** actuating element (7) is designed like a rocker.

4. The surgical instrument (1) according to one of claims 1 to 3, **characterized in that** the single swiveling movement of the actuating element (7) releases both a first connection between the rigid handle part (2) and the rigid part (22) and a second connection between the movable handle part (3) and the movable push rod (16) in a distal direction.

5. The surgical instrument (1) according to one of claims 1 to 4, **characterized in that** the actuating element (7) comprises an outwards facing actuating surface (19) which brings about the swiveling movement of the actuating element (7) via a pushing motion and in this way ensures the ability of the shaft section (5) to get released from the handle (4).

6. The surgical instrument (1) according to claim 5, **characterized in that** the actuating surface (19) extends substantially so as to be flush with an outer surface of the rigid handle part (2) in a locked condition of the handle (4) and the shaft section (5).

7. The surgical instrument (1) according to one of claims 1 to 6, **characterized in that** the actuating element (7) comprises a latching protrusion (9) which is prepared to establish a form fit with a receiving pocket (10) of the rigid part (22) in the locked condition.

8. The surgical instrument (1) according to one of claims 1 to 5, **characterized in that** the actuating element (7) in the locked condition is pre-tensioned by a leaf spring (12) formed by the rigid handle part (2).

9. The surgical instrument (1) according to one of claims 4 to 8, **characterized in that** the movable handle part (3) comprises a drive pin (13) which is prepared to establish a form fit between the movable handle part (3) and the push rod (16) via an indentation (18) in the push rod (16), in order to transfer a rotation of the movable handle part (3) into a translation of the push rod (16).

10. The surgical instrument (1) according to claim 9, **characterized in that** the actuating element (7) has a contact surface (21) which is prepared to be in contact with the drive pin (13) in such a manner that the latter can be released from the indentation (18) irrespectively of the position of the movable handle part (3).

11. The surgical instrument (1) according to one of claims 7 to 10, **characterized in that** the latching protrusion (9) engages in the receiving pocket (10) such that upon occurrence of the form fit an acoustic signal is produced as a result of the snap-in process, which signals the locked condition to a user.

12. The surgical instrument (1) according to one of the preceding claims, **characterized in that** the one actuating element (7) has a double effect on the rigid part (22) and the push rod (16).

13. The surgical instrument (1) according to claim 12, **characterized in that** the actuating element (7) has its distal end provided with a latching tab (9) projecting toward the rigid part (22) and proximal thereto a contact surface (21) resting on the drive pin (13), wherein, upon actuation of the actuating element (7), the latching tab (9) is disengaged from the rigid part (22) and the contact surface (21) partially slides along the drive pin (13).

## Revendications

1. Instrument chirurgical (1) comportant section de tige (5) composée d'une partie rigide (22) et d'une baguette de poussée mobile (16), laquelle section est reliée au niveau de sa section d'extrémité proximale à une poignée (4) qui présente une partie de poignée rigide (2) et une partie de poignée (3) mobile par rapport à celle-ci,
ainsi qu'un mécanisme de desserrement (6) qui est actionnable de sorte que la section de tige (5) et la poignée (4) peuvent être découplées l'une de l'autre,
**caractérisé en ce que** le mécanisme de desserrement (6) présente un seul élément d'actionnement (7) qui desserre au moyen de préférence d'un seul mouvement d'actionnement, de préférence un pivotement, une première liaison entre la partie de poignée rigide (2) et la partie rigide (22) de la section de tige (5) et une seconde liaison entre la partie de poignée mobile (3) et la baguette de poussée mobile (16) de la section de tige (5) de sorte que la section de tige (5) et la poignée (4) sont mobiles librement en tirant la section de tige (5) dans la direction distale par rapport à la poignée (4).

2. Instrument chirurgical (1) selon la revendication 1, **caractérisé en ce que** par le mouvement d'actionnement, de préférence le pivotement, de l'élément d'actionnement (7) la section de tige (5) et la poignée (4) sont desserrées entièrement l'un de l'autre dans la direction distale indépendamment de la position de la partie de poignée mobile (3) par rapport à la partie de poignée rigide (2).

3. Instrument chirurgical (1) selon l'une des revendications 1 ou 2, **caractérisé en ce que** l'élément d'actionnement (7) est réalisé de manière à basculer.

4. Instrument chirurgical (1) selon l'une des revendications 1 à 3, **caractérisé en ce que** par un pivotement de l'élément d'actionnement (7) aussi bien une première liaison entre la partie de poignée rigide (2) et la partie rigide (22) qu'une seconde liaison entre la partie de poignée mobile (3) et la baguette de poussée mobile (16) sont desserrées dans la direction distale.

5. Instrument chirurgical (1) selon l'une des revendications 1 à 4, **caractérisé en ce que** l'élément d'actionnement (7) présente une surface d'actionnement (19) tournée vers l'extérieur qui provoque le pivotement de l'élément d'actionnement (7) par l'intermédiaire d'un mouvement de pression et ainsi assure le desserrement de la section de tige (5) par la poignée (4).

6. Instrument chirurgical (1) selon la revendication 5, **caractérisé en ce que** la surface d'actionnement (19) s'étend dans un état de blocage entre la poignée (4) et la section de tige (5) pour l'essentiel à fleur avec une surface extérieure de la partie de poignée rigide (2).

7. Instrument chirurgical (1) selon l'une des revendications 1 à 6, **caractérisé en ce que** l'élément d'actionnement (7) présente une saillie d'enclenchement (9) qui est conçue pour adopter une complémentarité de formes à l'état de blocage avec une poche de réception (10) de la partie rigide (22).

8. Instrument chirurgical (1) selon l'une des revendications 1 à 5, **caractérisé en ce que** l'élément d'actionnement (7) est précontraint à l'état de blocage par l'intermédiaire d'un ressort à lames (12) formé par la partie de poignée rigide (2).

9. Instrument chirurgical (1) selon l'une des revendications 4 à 8, **caractérisé en ce que** la partie de poignée mobile (3) présente un entraîneur (13) qui est conçu pour générer par l'intermédiaire d'une encoche (18) dans la baguette de poussée (16) une complémentarité de formes entre la partie de poignée mobile (3) et la baguette de poussée (16) pour transformer une rotation de la partie de poignée mobile (3) en une translation de la baguette de poussée (16).

10. Instrument chirurgical (1) selon la revendication 9, **caractérisé en ce que** l'élément d'actionnement (7) présente une surface d'appui (21) qui est conçue pour être en contact avec l'entraîneur (13) de sorte que celui-ci peut être desserré de l'encoche (18) indépendamment de la position de la pièce de poignée mobile (3).

11. Instrument chirurgical (1) selon l'une des revendications 7 à 10, **caractérisé en ce que** la saillie d'enclenchement (9) se met en prise dans la poche de réception (10) de sorte que lors de l'établissement de la complémentarité de formes par l'encliquetage un signal acoustique apparaît, lequel signale l'état de blocage à un utilisateur.

12. Instrument chirurgical (1) selon l'une des revendications précédentes, **caractérisé en ce qu'**un élément d'actionnement (7) agit doublement sur la partie rigide (22) et la baguette de poussée (16).

13. Instrument chirurgical (1) selon la revendication 12, **caractérisé en ce que** l'élément d'actionnement (7) forme au niveau de son extrémité distale un ergot d'enclenchement (9) saillant dans la partie rigide (22) et forme à proximité de cet ergot une surface d'appui (21) reposant sur un entraîneur (13), dans lequel lors d'un actionnement de l'élément d'actionnement (7) l'ergot d'enclenchement (9) s'écarte de la partie rigide (22) et la surface d'appui (21) glisse partiellement au niveau de l'entraîneur (13).
